# EUROPÄISCHE PATENTSCHRIFT

(11) **EP 2 703 012 B1**
(45) Veröffentlichungstag und Bekanntmachung des Hinweises auf die Patenterteilung: **15.07.2015**
(21) Anmeldenummer: 13003888.8
(22) Anmeldetag: 05.08.2013
(51) Int. Cl.: A61L 2/14, A61L 2/20, B08B 9/08

(54) **Vorrichtung und Verfahren zur Desinfektion von Behältern**
Method and device for disinfecting containers
Dispositif et procédé destinés à la désinfection de récipients

(30) Priorität: 30.08.2012 DE 102012108042
(43) Veröffentlichungstag der Anmeldung: 05.03.2014
(73) Patentinhaber: Nonnenmacher, Klaus, Prof.Dipl.-Ing., 72070 Tübingen (DE)
(72) Erfinder: Nonnenmacher, Klaus, Prof.Dipl.-Ing., 72070 Tübingen (DE)
(74) Vertreter: Sacht-Gorny, Gudrun

(56) Entgegenhaltungen:
- WO-A1-2012/130197
- DE-A1- 3 502 242
- DE-A1- 19 542 447
- US-A- 3 383 163
- US-A1- 2004 140 269

## Beschreibung

Die vorliegende Erfindung betrifft eine Vorrichtung zur Desinfektion eines Innenraums eines Behälters. Ferner betrifft die vorliegende Erfindung ein Verfahren zur Desinfektion des Innenraums von Holzbehältern.

Die Desinfektion von Behältern, insbesondere in der Lebensmittelindustrie wird im Allgemeinen durch Desinfektion mit Flüssigkeiten und ggf. durch zusätzliche mechanische Behandlung durchgeführt. Daneben wird bei der Desinfektion von Holzfässern seit jeher Schwefeldioxidgas verwendet. Dazu wird z.B. Schwefelspan innerhalb eines Holzfasses verbrannt oder verkokelt.

Neben der direkten bioziden Wirkung von Schwefeldioxidgas erfolgt die biozide Wirkung von Sulfiten in wässriger Lösung, die z.B. durch Lösungsgleichgewichte zwischen der Gasphase und vorhandener (Rest)Feuchtigkeit an Oberflächen erhalten werden kann, vor allem durch die undissoziierte Form der schwefligen Säure (H₂SO₃). Damit diese eine optimale Wirkung erzielt, muss somit ein niedriger pH-Wert, vorzugsweise unterhalb von 3, vorliegen. Dies bedeutet in der Folge, dass diese für Lebensmittel verwendeten Behälter, insbesondere Holzfässer, nach dem Sterilisationsvorgang durch Reinigung mit sterilem Wasser von Abbauprodukten und Säureresten befreit werden müssen. Dies muss für eine umfassende Entfernung und Desinfektion gründlich geschehen und bedeutet somit einen hohen Zeitaufwand.

Die biozide Wirkung von Schwefeldioxid und den Sulfiten beruht insbesondere auf deren Reduktionswirkung. Dabei wird der zur Verfügung stehende Sauerstoff von Schwefeldioxid und von den Sulfiten schnell abgefangen, so dass aerobe Organismen in ihrem Stoffwechsel gehindert werden und somit absterben. Die biozide Wirkung gegenüber anaeroben Mikroorganismen ist hingegen schlecht.

Ferner führt die Verwendung von Schwefeldioxid zur Desinfektion von Holzfässern dazu, dass das Holz das desinfizierende Gas Schwefeldioxid leicht aufnimmt und selbst nach längerem Reinigen mit Wasser stets noch Schwefeldioxidreste im Holz vorhanden sind. Diese werden nach dem Einfüllen von Lebensmitteln, wie z.B. von Wein, nach und nach an dieses Lebensmittel abgegeben, was zu überhöhten Sulfitgehalten im Lebensmittel führen kann. In Anbetracht der bioziden Wirkung der Sulfite stellt dies eine unerwünschte gesundheitliche Belastung dar. Ungebundene Sulfite sind dafür bekannt, dass sie pseudoallergische sowie anaphylaktische Reaktionen nach inhalativer und oraler Aufnahme verursachen. Ferner sind Sulfite haut- und schleimhautreizend.

Aus der US-Patentschrift US 3 383 163 A ist ein Verfahren zur Desinfektion der inneren Oberflächen einer Flasche bekannt, bei dem außen um die Flasche ein elektrischer Leiter angelegt wird. Im Inneren der Flasche befindet sich eine Erdungselektrode. In die Flasche wird ein Inertgas eingebracht, bevor ein hoher elektrischer Strom an den elektrischen Leiter angelegt wird. Auf diese Weise wird kurzfristig ein Plasma in der Flasche erzeugt, wodurch eine Sterilisierung der inneren Oberflächen bewirkt werden soll.

Aus der deutschen Offenlegungsschrift DE 35 02 242 A1 geht ein Verfahren zum Sterilisieren von Fässern hervor, bei dem Ozongas oder Ozonwasser in die Fässer eingeleitet wird.

Der vorliegenden Erfindung lag die Aufgabe zugrunde, eine Vorrichtung und ein Verfahren bereitzustellen, die eine Desinfektion von Behältern und insbesondere von Holzfässern gestatten, ohne dass dabei eine aufwändige mechanische Reinigung bzw. Nachreinigung notwendig ist und insbesondere ohne dass dabei Rückstände in den Behältern verbleiben, die zu einer Belastung der Lebensmittel führen, die sich negativ auf die Gesundheit von Konsumenten der Lebensmittel auswirken kann. Ferner soll dabei die Desinfektionswirkung gegenüber den bekannten Methoden nicht verschlechtert und vorzugsweise sogar verbessert werden.

Die Aufgabe wird zum einen gelöst durch eine Vorrichtung zur Desinfektion eines Innenraums eines Behälters, vorzugsweise eines Holzbehälters, mit
- einem stabförmigen Gehäuse, mit einem ersten Ende zur Anordnung innerhalb des Behälters, einem gegenüberliegenden zweiten Ende und einem inneren Hohlraum,
- einer Ozonerzeugungseinheit im inneren Hohlraum, und
- zumindest einer Mehrzahl von Öffnungen im Gehäuse im Bereich des ersten Endes.
Weiterhin weist das Gehäuse an seinem zweiten Ende außen ein Dichtungselement auf, durch das die Vorrichtung dichtend in einer Öffnung des Behälters aufgenommen werden kann.

Wenn hier oder im Folgenden von "im Bereich" des ersten oder zweiten Endes die Rede ist, bezieht sich das auf den Abschnitt des Gehäuses, der der Hälfte, vorzugsweise dem Drittel des Gehäuses entspricht, die/das dem jeweiligen Ende zugewandt ist.

Ozon zur Verwendung als Desinfektionsmittel hat den Vorteil, dass hierdurch aerobe als auch anaerobe Mikroorganismen gleichermaßen vollständig abgetötet werden. Hierdurch können selbst bei pH-Werten um 7, das heißt im neutralen Milieu, Tötungsraten erzielt werden, die keine nachweisbaren Restkeimgehalte mehr hinterlassen. Ozon dringt als Gas in jede Ecke des Behälters und kann sogar bei der Verwendung in Holzbehältern in das Holz eindringen und somit zu einer optimalen Desinfektion des gesamten Behälters führen. Daneben ist durch die Ozonerzeugungseinheit der Vorrichtung die Möglichkeit gegeben, das Ozon innerhalb des Behälters bereitzustellen. Die Ozonerzeugungseinheit ist dazu in dem stabförmigen Gehäuse angeordnet. Dieses stabförmige Gehäuse gestattet es, die gesamte Vorrichtung durch eine gewöhnliche, bereits vorhandene Öffnung des Behälters, z.B. eine Ein- bzw. Ausgießöffnung, in den Behälter einzusetzen. Dadurch gelangt das hier als erstes Ende bezeichnete Ende in den Behälter und führt dazu, dass durch die Ozonerzeugungseinheit bereitgestelltes Ozon durch die Mehrzahl von Öffnungen im Bereich des ersten Endes in den Innenraum des Behälters diffundieren kann. Dazu ist die Ozonerzeugungseinheit vorzugsweise auch im Bereich der Mehrzahl von Öffnungen angeordnet. Als Trägergas für das verwendete Ozon kann somit das in dem Behälter bereits vorhandene Gas, in der Regel Luft, verwendet werden. Dieses diffundiert durch die Mehrzahl von Öffnungen zu der Ozonerzeugungseinheit, woraufhin der in der Luft vorhandene Luftsauerstoff an dieser Ozonerzeugungseinheit in Ozon umgewandelt werden kann. Eine Zufuhr von Ozon mittels einer außerhalb des Behälters liegenden Ozonerzeugungseinheit ist somit nicht notwendig und eine Gefährdung für die Gesundheit der Benutzer dieser erfindungsgemäßen Vorrichtung ist somit nicht gegeben. Das Ergebnis ist somit eine vollständige Desinfektion des Behälters und ferner auch eine Beseitigung von unerwünschten und störenden Geruchs- und Geschmacksträgern durch die Oxidation mit Ozon. Durch die kompakte Anordnung mit der Ozonerzeugungseinheit im Gehäuse und der einfachen Einführbarkeit in einen zu desinfizierenden Behälter durch bereits vorhandene Öffnungen, weist die erfindungsgemäße Vorrichtung zusätzlich eine einfache Handhabbarkeit auf.

In einer erfindungsgemäßen Ausgestaltung der Vorrichtung weist das Gehäuse einen runden Querschnitt auf.

Diese Gehäuseform hat den Vorteil, dass die erfindungsgemäße Vorrichtung somit einfach in die üblicherweise als runde Öffnungen in den jeweiligen Behältern, wie z.B. in Holzfässern, vorgesehenen Öffnungen eingeführt und eingesetzt werden kann.

In einer weiteren Ausgestaltung der erfindungsgemäßen Vorrichtung ist das Gehäuse aus Metall.

Die Verwendung von Metall, vorzugsweise von Edelstahl, hat den Vorteil, dass dadurch ein Material für das Gehäuse verwendet wird, welches zum einen nicht selber für das Desinfektionsmittel Ozon anfällig ist und somit nicht von diesem beschädigt wird, und das gleichzeitig ein Material darstellt, welches auf einfache Weise selber gereinigt und ggf. desinfiziert werden kann.

Erfindungsgemäß weist das Gehäuse an seinem zweiten Ende außen ein Dichtungselement auf, durch das die Vorrichtung dichtend in eine Öffnung des Behälters aufgenommen werden kann.

Durch diese Ausgestaltung ist es möglich, dass die Vorrichtung in dichtender Weise in einen Behälter, wie z.B. in ein Holzfass, eingesetzt werden kann. Die Ozonbereitstellung bzw. -erzeugung erfolgt dann innerhalb des Behälters, der, vorausgesetzt, er weist ansonsten auch keine weiteren offenen Öffnungen auf, einen komplett von der äußeren Umgebung abgeschlossenen Hohlraum darstellt, in dem eine vorzugsweise kontinuierliche Ozonerzeugung erfolgt, ohne dass Ozon nach außen treten kann. Dies vermindert weiter die Gefährdung von den jeweiligen Benutzern der erfindungsgemäßen Vorrichtung und im Allgemeinen von der sonstigen Umgebung. Nach Abschluss der Desinfektion wird die Ozonerzeugung eingestellt und nach einer gewissen Wartezeit, in der das restliche, im Behälter vorhandene Ozon zerfallen ist, die Vorrichtung wieder aus der Öffnung des Behälters entfernt. So wird eine gesundheitliche Gefährdung der Umgebung komplett ausgeschlossen.

In einer weiteren Ausgestaltung der erfindungsgemäßen Vorrichtung ist die Mehrzahl von Öffnungen im Gehäuse als Rundlöcher ausgestaltet.

Eine Ausgestaltung der Mehrzahl von Öffnungen als Rundlöcher hat den Vorteil, dass diese vergleichsweise einfach in das Gehäuse eingebracht werden können und im Übrigen auf bereits bekannte Methoden zur Herstellung solcher Gehäuse zurückgegriffen werden kann.

In einer alternativen Ausgestaltung der erfindungsgemäßen Vorrichtung ist die Mehrzahl von Öffnungen im Gehäuse als Längsschlitze ausgestaltet.

Die Verwendung von Längsschlitzen hat den Vorteil, dass hierbei eine vergleichsweise große Öffnung bereitgestellt werden kann, die einen einfachen und effektiven Transport von Luftsauerstoff in die Vorrichtung sowie von Ozon aus der Vorrichtung hinaus, z.B. durch Diffusion, ermöglicht. Daneben können diese Öffnungen jedoch auch zusätzlich so bereitgestellt werden, dass die strukturelle Integrität des gesamten stabförmigen Gehäuses nicht negativ beeinflusst wird. Die Längsschlitze können dabei mit Bezug auf die Längsrichtung des stabförmigen Gehäuses quer, längs und auch schräg zu dieser verlaufend im Gehäuse angeordnet sein. Vorzugsweise verlaufen die Längsschlitze quer oder schräg zu dieser und besonders bevorzugt quer zur Längserstreckung.

In einer weiteren Ausgestaltung der erfindungsgemäßen Vorrichtung weist die Ozonerzeugungseinheit eine Induktionselektrode und eine Entladungselektrode auf, die durch ein festes Dielektrikum voneinander getrennt sind.

Eine solche Ozonerzeugungseinheit hat den Vorteil, dass sie Ozon bereitstellen kann, indem sie mit einer geringen Spannung und einem geringen Stromverbrauch, z.B. bei 12 V und mit weniger als 500 mA, betrieben werden kann. Dennoch wird hierbei eine effektive Menge an Ozon bereitgestellt, die bspw. im Bereich zwischen 10 und 50 mg O₃/h liegt. Ferner wird aufgrund der Wärmeentwicklung an der außen liegenden Entladungselektrode bzw. an dem zwischen den Elektroden liegenden Dielektrikum eine Konvektion und somit ein Luftstrom erzeugt. Dies begünstigt zusätzlich das Einströmen von Luftsauerstoff von außerhalb der Vorrichtung in das Gehäuse der Vorrichtung sowie den Abtransport von erzeugtem Ozon aus dem inneren Hohlraum des Gehäuses der Vorrichtung nach außen in den Innenraum des Behälters, z.B. in den Innenraum des Holzfasses.

In einer alternativen Ausgestaltung der erfindungsgemäßen Vorrichtung weist die Ozonerzeugungseinheit eine erste spitzförmige Elektrode und eine zweite Elektrode auf, wobei die zweite Elektrode durch das Gehäuse gebildet wird und das Gehäuse in Längsrichtung gesehen auf Höhe der spitzförmigen Elektrode zumindest eine Öffnung aufweist.

Diese Ausgestaltung der Ozonerzeugungseinheit hat den Vorteil, dass hierbei ein Elektronenspray erzeugt wird, der ausgehend von der spitzförmigen Elektrode in Richtung der zumindest einen Öffnung im Gehäuse an den Rand dieser Öffnung(en) führt. Aufgrund der kinetischen Energie der Elektronen, sowie auch der ggf. entstandenen Ionen und Radikale, werden diese zur Öffnungskante im Gehäuse hin beschleunigt und können so aus dem Gehäuse in den Innenraum des Behälters gelangen. Dadurch wird die Konvektion innerhalb des Gehäuses der Vorrichtung verstärkt und ist dabei von der spitzförmigen Elektrode weggerichtet. Dies führt u.a. dazu, dass die Bildung von Kondensaten, die im Entladungsbereich, dem so genannten Plasma, von anderen Ozonerzeugungseinheiten entstehen können, vermieden wird.

In einer weiteren Ausgestaltung der erfindungsgemäßen Vorrichtung weist die Vorrichtung ferner eine UV-Lichtquelle auf, die im Gehäuse angeordnet ist.

Das Vorsehen einer UV-Lichtquelle hat den Effekt, dass aus dem erzeugten Ozon zusammen mit bspw. im Innenraum des Behälters vorhandenem Wasser(dampf) unter Einwirkung der UV-Strahlung aus der UV-Lichtquelle Peroxide, insbesondere Wasserstoffperoxid, gebildet werden können. Diese haben zum einen die Fähigkeit, selber zur Desinfektion des Behälters beizutragen und können ferner als Ausgangs- bzw. Startsubstanz für radikalische Kettenreaktionen dienen. Diese radikalischen Kettenreaktionen werden wiederum durch die von der UV-Lichtquelle emittierte UV-Strahlung initiiert und gegebenenfalls auch aufrecht erhalten. Vorzugsweise weist das Gehäuse der Vorrichtung in Längsrichtung gesehen auf Höhe der UV-Lichtquelle keine Öffnungen auf, so dass das UV-Licht hier nicht unmittelbar aus dem inneren Hohlraum des Gehäuses austreten kann, sondern zunächst an der Gehäuseinnenwand reflektiert und somit verstärkt wird. Neben diesen Effekten stellt die UV-Lichtquelle ferner eine Wärmequelle dar, die zusätzlich die zuvor schon erwähnte Konvektion des Luftsauerstoffs in das Gehäuse und des gebildeten Ozons aus dem Gehäuse verstärkt. In bevorzugten Ausgestaltungen kann das Gehäuse ferner so ausgestaltet sein, dass eine Aufnahme von Wasser im ersten Ende des Gehäuses möglich ist, welches durch die Konvektion und/oder Wärme verdampft und somit die zuvor bereits erläuterte Peroxidbildung begünstigt. Die Wasserstoffperoxidbildung kann weiterhin durch einen Wasserverdampfer verstärkt werden. Der Wasserverdampfer kann beispielsweise separat im Innenraum des Behälters angeordnet sein oder in die erfindungsgemäße Vorrichtung integriert sein.

In einer weiteren Ausgestaltung der erfindungsgemäßen Vorrichtung weist die Vorrichtung zusätzlich oder alternativ eine UV-Lichtquelle auf, die außen am Gehäuse angeordnet ist.

Das Vorsehen einer UV-Lichtquelle außen am Gehäuse der Vorrichtung, vorzugsweise am ersten Ende, führt dazu, dass zunächst Ozon innerhalb des Gehäuses gebildet werden kann ohne gleich aufgrund der Einwirkung von UV-Strahlung zusammen mit Wasser zu Wasserstoffperoxid umgesetzt zu werden. Diese Reaktion tritt aufgrund der äußeren Anordnung der UV-Lichtquelle erst dann auf, wenn das Ozon durch Diffusion und die zuvor erwähnte Konvektion aus dem inneren Hohlraum des Gehäuses nach außen in den Innenraum des Behälters gelangt ist.

In einer bevorzugten Ausgestaltung der Vorrichtung besteht das Gehäuse und vor allem der Boden der Vorrichtung, also insbesondere das Gehäuse im Bereich des ersten Endes, zumindest teilweise aus einem inerten Material, beispielsweise Edelstahl, Kunststoff, Aluminium, Silber, Palladium oder anderes. Es können auch Kombinationen dieser Materialien eingesetzt werden. Hierdurch kann die Stabilität und Lebensdauer der Vorrichtung weiter verbessert und verlängert werden. Zudem erleichtert ein inertes Material die Reinigung und Desinfektion der Vorrichtung.

Weiterhin kann das Gehäuse im Bereich des ersten Endes zumindest teilweise ein Metalloxidmaterial aufweisen, insbesondere eine oxidische Schicht oder Beschichtung. Hierdurch werden katalytische Effekte bei der Umsetzung des Ozons erzielt, die die Wirksamkeit der erfindungsgemäßen Vorrichtung verstärken. Besonders geeignet ist beispielsweise eine Beschichtung oder ein oxidischer Überzug aus Aluminiumoxid und/oder Kupferoxid und/oder Manganoxid. Die Beschichtung oder der Überzug kann sich beispielsweise auf oder an dem unteren Abschluss des Gehäuses, insbesondere an der Bodenkappe oder an dem Bodendeckel befinden. Die oxidische Beschichtung oder der Überzug kann innen und/oder außen angebracht sein. Die Fläche des Bodendeckels ist im Allgemeinen ausreichend, um eine sehr effektive Unterstützung der Wirksamkeit der Vorrichtung durch den katalytischen Effekt des oxidischen Materials zu erreichen. Zudem ist insbesondere der Bodendeckel für eine Ausstattung mit dem oxidischen Material sehr geeignet, da dann das übrige Gehäuse mit einer inerten Oberfläche, zum Beispiel einer Edelstahloberfläche, ausgestattet sein kann.

Die eingangs genannte Aufgabe wird ferner durch ein Verfahren zur Desinfektion des Innenraums von Holzbehältern durch Bereitstellen von Ozon im Holzbehälter gelöst.

Entsprechend der zuvor gemachten Ausführungen hat die Desinfektion der Holzbehälter, insbesondere von Weinfässern, mit Ozon den Vorteil, dass auf die bisher verwendete Desinfektion mithilfe von Schwefeldioxid verzichtet werden kann. Dadurch wird die bereits diskutierte Gesundheitsgefährdung durch die Lebensmittel, die mit den erhöhten Sulfitgehalten belastet sein können, reduziert. Ferner ist die Desinfektionswirkung des Ozons auch bei anaeroben Mikroorganismen gegeben, so dass sogar die Desinfektion als solche mit Ozon als Desinfektionsmittel gegenüber der Schwefeldioxiddesinfektion verbessert ist.

In einer weiteren Ausgestaltung des erfindungsgemäßen Verfahrens wird das Ozon im Holzbehälter erzeugt.

Durch die Erzeugung von Ozon direkt im Holzbehälter ist eine externe Erzeugung von Ozon mit weiteren Zuleitungen in den Behälter unnötig. Dies verringert das Risiko des Austritts von Ozon auf dem Weg in den Holzbehälter. Dadurch wird die Gefährdung der Anwender des erfindungsgemäßen Verfahrens durch Ozon bzw. Ozonlecks deutlich reduziert.

Das erfindungsgemäße Verfahren weist insbesondere die folgenden Schritte auf:
- Anordnen einer Ozonerzeugungseinheit im Holzbehälter,
- Verschließen des Holzbehälters,
- Erzeugen von Ozon durch die Ozonerzeugungseinheit.

Durch diese erfindungsgemäßen Verfahrensschritte wird das zuvor schon erwähnte vorteilhafte Erzeugen des Ozons im Holzbehälter umgesetzt. Dazu wird der Holzbehälter zusätzlich nach dem Anordnen einer Ozonerzeugungseinheit im Holzbehälter verschlossen. So wird nicht nur die Gefährdung von Anwendern des erfindungsgemäßen Verfahrens durch Ozonlecks in Zuleitungen eliminiert, sondern auch eine Gefährdung durch austretendes Ozon aus dem Holzbehälter vermieden. Das Ozon wird somit in der abgeschlossenen Atmosphäre innerhalb des Holzbehälters erzeugt, wodurch eine Desinfektion über einen definierten Zeitraum innerhalb des Holzbehälters und somit auch an dessen Innenwandungen stattfindet.

In einer weiteren Ausgestaltung des erfindungsgemäßen Verfahrens erfolgt das Erzeugen von Ozon kontinuierlich.

Die kontinuierliche Erzeugung von Ozon hat den Vorteil, dass so auch mit möglicherweise geringen Mengen an erzeugtem Ozon über einen vorgegebenen Zeitraum eine ozonhaltige Atmosphäre im Holzbehälter aufrechterhalten wird. Dies führt dazu, dass entsprechend vorhandene Mikroorganismen im Holzbehälter innerhalb dieses Zeitraums nach und nach abgetötet werden. Nach Beenden der Ozonerzeugung bleibt der Holzbehälter vorzugsweise noch einige Zeit verschlossen, bis das restliche, im Holzbehälter noch vorhandene Ozon abgebaut ist. Die Desinfektion mit Ozon ist damit rückstandsfrei und etwaige nachfolgende Gesundheitsbelastungen durch das Desinfektionsmittel sind ausgeschlossen.

In einer weiteren Ausgestaltung des erfindungsgemäßen Verfahrens wird der Innenraum des Holzbehälters ferner UV-Licht ausgesetzt.

Das Aussetzen des Innenraums mit UV-Licht hat den Vorteil, dass so aus dem Ozon zusammen mit noch vorhandenem Wasser in der Atmosphäre des Innenraums des Holzbehälters Wasserstoffperoxid gebildet werden kann. Dieses reagiert ebenfalls als Desinfektionsmittel auf die im Holzbehälter vorhandenen Mikroorganismen. Auch Wasserstoffperoxid hat den Vorteil, dass es rückstandsfrei abgebaut wird und somit keine weitere gesundheitliche Belastung von diesem Desinfektionsmittel ausgeht. Ferner kann UV-Licht zum rascheren Abbau vom restlichen Ozon nach der Ozonerzeugung eingesetzt werden.

In einer weiteren Ausgestaltung des erfindungsgemäßen Verfahrens wird im Innenraum ferner Wasserdampf bereitgestellt.

Durch die Bereitstellung von zusätzlichem Wasserdampf wird die zuvor erwähnte Bildung von Wasserstoffperoxid durch das UV-Licht zusätzlich begünstigt. Die Erzeugung des Wasserdampfes kann dabei durch einen separaten Wasserverdampfer im Innenraum des Holzbehälters erfolgen oder auch durch die gleiche Vorrichtung, die auch das Ozon bereitstellt, geschehen.

Durch die Bereitstellung von metalloxidischem Material im Bereich des Bodens der Vorrichtung, die für das Verfahren verwendet werden kann, also insbesondere im Bereich des ersten Endes des Gehäuses, werden vorteilhafte katalytische Effekte bei der Umsetzung des Ozons erzielt, die die Wirksamkeit des erfindungsgemäßen Verfahrens verstärken. Besonders geeignet ist eine oxidische Beschichtung oder ein oxidischer Überzug, beispielsweise aus Aluminiumoxid und/oder Kupferoxid und/oder Manganoxid. Weiterhin kann das Gehäuse selbst zumindest teilweise aus einem inerten Material bestehen, beispielsweise Edelstahl, Kunststoff, Aluminium, Silber, Palladium oder anderes. Es können auch Kombinationen dieser Materialien eingesetzt werden. Hierdurch können die Stabilität und Lebensdauer sowie die Handhabbarkeit der Vorrichtung weiter verbessert und verlängert werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:
- Fig. 1: eine schematische Seitenansicht eines ersten Ausführungsbeispiels einer erfindungsgemäßen Vorrichtung,
- Fig. 2: eine schematische Seitenansicht eines zweiten Ausführungsbeispiels einer erfindungsgemäßen Vorrichtung,
- Fig. 3: eine schematische Seitenansicht der Vorrichtung aus Fig. 2 entsprechend einer Blickrichtung gemäß Pfeil III,
- Fig. 4: eine ausschnittsweise vergrößerte schematische Schnittansicht der Vorrichtung aus den Figuren 2 und 3 entsprechend der Linie IV-IV aus Fig. 3,
- Fig. 5: eine weitere ausschnittsweise vergrößerte Schnittansicht eines dritten Ausführungsbeispiels einer erfindungsgemäßen Vorrichtung analog zur Darstellung von Fig. 4,
- Fig. 6: eine ausschnittsweise schematische Schnittansicht eines vierten Ausführungsbeispiels einer erfindungsgemäßen Vorrichtung analog zur Darstellung von Fig. 4,
- Fig. 7: eine ausschnittsweise schematische Schnittansicht eines fünften Ausführungsbeispiels einer erfindungsgemäßen Vorrichtung analog zur Darstellung von Fig. 6,
- Fig. 8: ein Holzgefäß/-fass in einer schematischen perspektivischen Seitenansicht mit einer erfindungsgemäßen Vorrichtung entsprechend der Fig. 2, und
- Fig. 9: eine schematische Schnittansicht des Holzgefäßes aus Fig. 8.

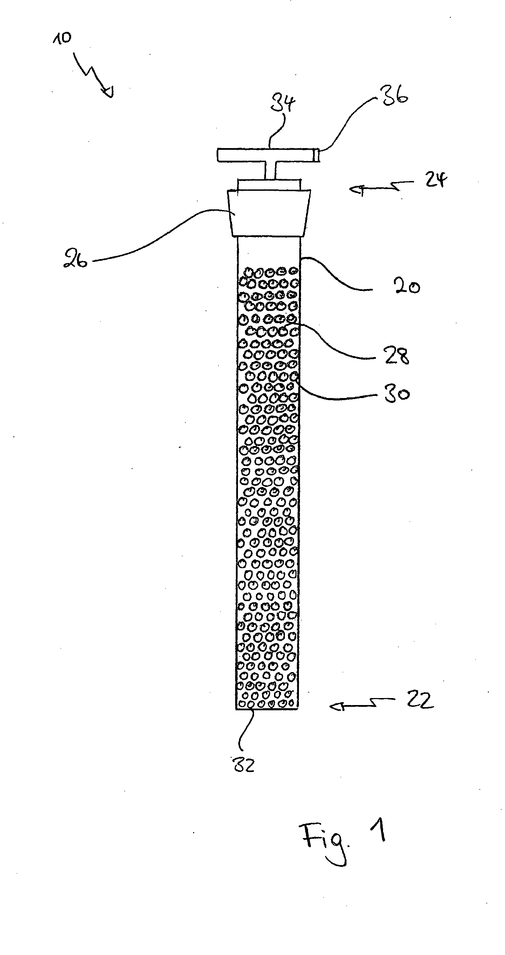

Beispielhafte Ausführungsformen der erfindungsgemäßen Vorrichtung sind in den Figuren 1 bis 9 gezeigt und anhand dieser erläutert und jeweils in ihrer Gesamtheit mit den Bezugsziffern 10, 12, 14, 16 und 18 bezeichnet. Dabei sind identische Merkmale unter den jeweiligen Ausführungsformen jeweils mit den identischen Bezugsziffern bezeichnet.

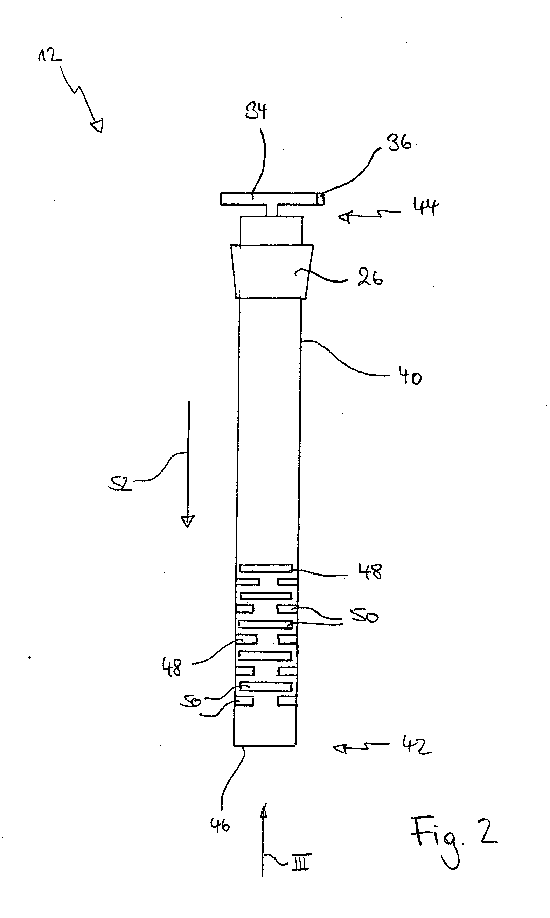

Die Vorrichtung 10 in Fig. 1 weist ein stabförmiges Gehäuse 20 mit einem ersten Ende 22 und einem zweiten Ende 24, gegenüberliegend vom ersten Ende 22, auf. Die Vorrichtung 10 weist ferner einen inneren Hohlraum im Gehäuse 20 auf, in dem eine Ozonerzeugungseinheit angeordnet ist. Diese Ozonerzeugungseinheit ist hier nicht näher in Fig. 1 und im Rahmen der Vorrichtung 10 gezeigt, wird aber im Folgenden im Zusammenhang mit dem Ausführungsbeispiel der Vorrichtung 12 und den Figuren 2 ff. näher und beispielhaft beschrieben.

Im Bereich des zweiten Endes 24 weist die Vorrichtung 10 ein Dichtungselement 26 auf. Dieses Dichtungselement 26 ist in dem vorliegenden Ausführungsbeispiel konisch ausgestaltet, wobei es in der Richtung vom zweiten Ende 24 zum ersten Ende 22 hin schmaler wird. Das Dichtungselement 26 kann vorzugsweise aus einem elastischen Material hergestellt sein. Dieses elastische Material ist vorzugsweise ein gummiartiger Stoff, der so ausgewählt ist, dass er nicht oder nur wenig anfällig für Ozon ist. Nicht einschränkende Beispiele für solche gummiartigen Substanzen sind z.B. synthetische Kautschuks, wie bspw. Ethylen-Propylen-Dien-Kautschuk (EPDM), und besonders bevorzugt Silikone bzw. Silikongummis. Alternativ können auch nicht-elastische Dichtungselemente verwendet werden, die ebenfalls vorzugsweise aus ozonresistenten Substanzen bestehen. Beispiele hierfür können Dichtungselemente aus Edelstahl oder vorzugsweise Polytetrafluorethylen (Teflon ®) sein.

Vom zweiten Ende 24 zum ersten Ende 22 hin gesehen ist hinter dem Dichtungselement 26 eine Vielzahl von Öffnungen 28 im Gehäuse 20 der Vorrichtung 10 angeordnet. Diese Öffnungen 28 sind vorliegend als Rundlöcher 30 ausgestaltet. Die Öffnungen 28 bzw. Rundlöcher 30 erstrecken sich dabei bis zum ersten Ende 22. Wenngleich aufgrund der Art der Darstellung in der Fig. 1 nur eine Seite der Vorrichtung 10 gezeigt ist, so sind die Öffnungen 28 umfänglich am Gehäuse 20, also auch auf der nicht gezeigten Rückseite der Vorrichtung 10, angeordnet.

Das Gehäuse 20 der Vorrichtung 10 ist im Übrigen als ein rohrförmiger Stab ausgebildet, weist also einen runden Querschnitt auf. Am ersten Ende 22 ist dieses Rohr, also das Gehäuse 20, mit einem Deckel 32 verschlossen. Dieser Deckel 32 kann entweder ein aus durchgehendem Material komplett verschlossener Deckel sein, oder aber auch Öffnungen, die bspw. vergleichbar zu den Rundlöchern 30 sind, aufweisen.

Am zweiten Ende 24 weist das Gehäuse einen Griff 34 auf. Dieser Griff 34 dient zum einen zum Halten der Vorrichtung 10 mit der Hand beim Einsetzen oder Herausnehmen der erfindungsgemäßen Vorrichtung 10 in einen zu desinfizierenden Behälter. Ferner weist der Griff 34 noch einen Stromanschluss 36 auf, über den die elektrische Stromversorgung der im Gehäuse 20 angeordneten Ozonerzeugungseinheit gewährleistet werden kann. Hierzu wird ein stromführendes Kabel an diesen Stromanschluss 36 eingesteckt. Der Stromanschluss 36 weist hierzu gängige Verbindungseinrichtungen, wie z.B. Buchsen, auf.

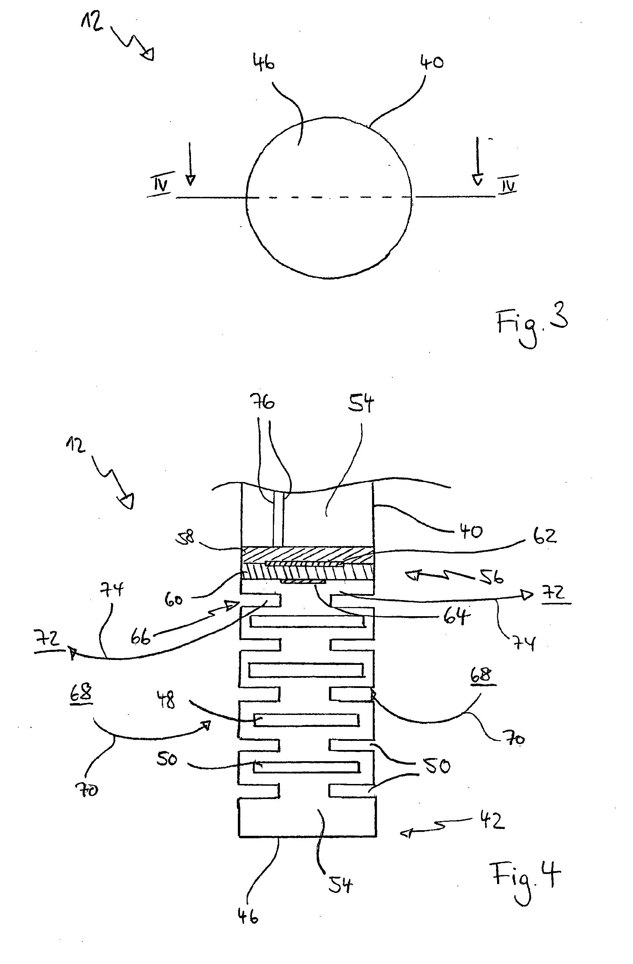

Das zweite Ausführungsbeispiel der Vorrichtung 12 in Fig. 2 weist ebenfalls ein Gehäuse 40 auf. Dieses Gehäuse 40 hat ebenfalls ein erstes Ende 42 sowie ein zweites Ende 44. Auch das Gehäuse 40 der Vorrichtung 12 ist stabförmig ausgestaltet und hat einen runden Querschnitt, wie dies insbesondere in der Darstellung der Fig. 3 zu sehen ist. Fig. 3 zeigt hier die Seite der Vorrichtung 12 am ersten Ende 42 des Gehäuses 40, die mit einem Deckel 46 verschlossen ist.

Am zweiten Ende 44 weist die Vorrichtung ebenso wie die Vorrichtung 10 einen Griff 34 mit einem Stromanschluss 36 auf. Ferner befindet sich im Bereich des zweiten Endes 44 ein Dichtungselement 26.

Während die Öffnungen 28, also die Rundlöcher 30, im Ausführungsbeispiel der Vorrichtung 10 aus Fig. 1 über nahezu die gesamte Länge des Gehäuses 20 erstreckt sind, weist die Vorrichtung 12 Öffnungen 48 auf, die lediglich im Bereich des ersten Endes 42 im Gehäuse 40 angeordnet sind. Hierbei meint eine Anordnung im Bereich des ersten Endes 42, dass in Anbetracht der gesamten Länge des Gehäuses 40 die entsprechenden Öffnungen hier erst nach der Hälfte, vorzugsweise im letzten Drittel, der Strecke zwischen zweitem Ende 44 und erstem Ende 42 auftreten und somit in Richtung auf das erste Ende 42 hin im Gehäuse 40 vorliegen.

Die Öffnungen 48 in der Vorrichtung 12 sind hier als Längsschlitze 50 ausgestaltet. Die Längsschlitze 50 sind in der Vorrichtung 12 so ausgestaltet, dass sie quer zur Längserstreckung des Gehäuses 40, die durch einen Pfeil 52 angedeutet ist, verlaufen. Entsprechend den zuvor gemachten Ausführungen im Zusammenhang mit der Vorrichtung 10 in Fig. 1 verlaufen auch die hier gezeigten Längsschlitze 50 umfänglich um das Gehäuse 40 herum und sind somit auch in vergleichbarer Weise auf der hier nicht gezeigten Rückseite der Vorrichtung 12 angeordnet. Der Ausdruck "Rückseite" bezieht sich hierbei auf die vom Betrachter abgewandte Seite der Darstellung der Fig. 2. Dies gilt ebenso für die zuvor gemachten Ausführungen im Zusammenhang mit der Vorrichtung 10 in Fig. 1.

In Fig. 4 ist ein Schnitt durch die Vorrichtung 12 im Bereich des ersten Endes 42 dargestellt. Hier ist zu erkennen, dass die Vorrichtung 12, bzw. das Gehäuse 40, einen inneren Hohlraum 54 aufweist. In diesem ist ebenfalls im Bereich des ersten Endes 42 eine Ozonerzeugungseinheit 56 angeordnet. In diesem speziellen Ausführungsbeispiel der Vorrichtung 12 ist die Ozonerzeugungseinheit 56 an dem Ende der Ansammlung der Öffnungen 48 angeordnet, das dem zweiten Ende 44 zugewandt ist. Mit Bezug auf die Darstellung der Figuren 2 und 4 findet sich die Ozonerzeugungseinheit 56 somit oberhalb der Öffnungen 48.

Die Ozonerzeugungseinheit 56 weist in diesem Ausführungsbeispiel zwei Keramikhälften 58 und 60 auf. Zwischen diesen Keramikhälften 58 und 60 ist eine Induktionselektrode 62 angeordnet. Auf der gegenüberliegenden Seite der Keramikhälfte 60 und im hier gezeigten Beispiel zu den Öffnungen 50 hin ist eine Entladungselektrode 64 auf der Keramikhälfte 60 angeordnet. Somit sind die Induktionselektrode 62 und die Entladungselektrode 64 durch ein Dielektrikum, in diesem Ausführungsbeispiel die Keramikhälfte 60, voneinander getrennt. Zum Zwecke der Isolierung kann die äußere Entladungselektrode 64 durch eine weitere Schicht abgedeckt sein, die hier nicht näher im Detail gezeigt ist.

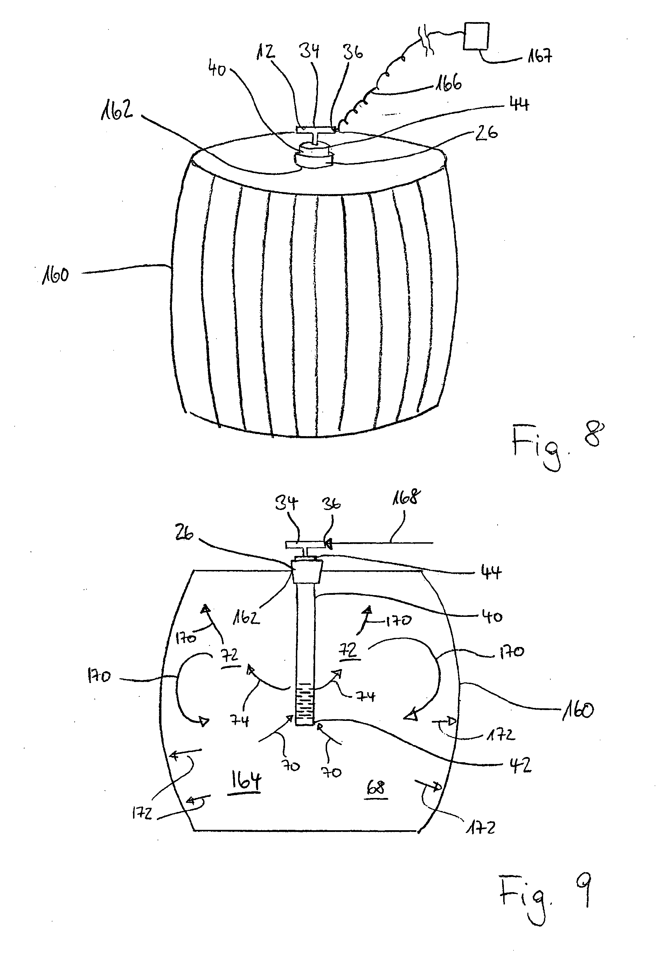

Um die Entladungselektrode 64 herum entsteht den Öffnungen 48 zugewandt eine Entladungszone 66. Aufgrund der Spannung zwischen der Induktionselektrode 62 und der Entladungselektrode 64 entsteht ferner ein elektrischer Stromfluss, der wiederum für eine Erwärmung der Keramikhälfte 60 sorgt. Durch diese Erwärmung der Keramikhälfte 60 entsteht ferner eine Konvektion der die Entladungselektrode 64 umgebenden Luft. So kann bspw. durch die Öffnungen 48, also im vorliegenden Ausführungsbeispiel der Vorrichtung 12 durch die Längsschlitze 50 sauerstoffhaltige Luft 68 in den Bereich des ersten Endes 42 in das Gehäuse 40 eindringen, wie dies durch Pfeile 70 verdeutlicht ist. Diese zuströmende O₂-haltige Luft 68 wird dann an der Entladungselektrode 64 im Bereich der Entladungszone 66 umgesetzt. Bei dieser Umsetzung werden die Sauerstoffmoleküle aufgrund der Spannung in der Entladungszone 66 aufgebrochen, wobei atomarer Sauerstoff sowie dreiatomiger Sauerstoff, also Ozon, entsteht. Diese dadurch gebildete ozonhaltige Luft 72 wird dann aufgrund der zuvor genannten Konvektion aus dem inneren Hohlraum 54 des Gehäuses 40 wieder nach außen befördert. Dies ist durch Pfeile 74 angedeutet. Die weitere Wirkweise der ozonhaltigen Luft 72 wird später im Zusammenhang mit den Figuren 8 und 9 noch näher erläutert.

Um eine entsprechende Stromversorgung der Ozonerzeugungseinheit 56 zu erhalten, sind Stromleiter, also Kabel 76, im inneren Hohlraum 54 des Gehäuses 40 zu der Ozonerzeugungseinheit 56 zugeführt. Diese Kabel 76 enden vorzugsweise im Bereich des zweiten Endes 44 an dem Stromanschluss 36 des Griffs 34.

Um eine zuvor bereits erwähnte Konvektion der Luft in den inneren Hohlraum 54 zu verstärken, kann in einer nicht näher gezeigten alternativen Ausführungsform der Deckel 46 durch einen Ventilator ersetzt werden. Der Ventilator ist damit am ersten Ende 42 des Gehäuses 40 angeordnet.

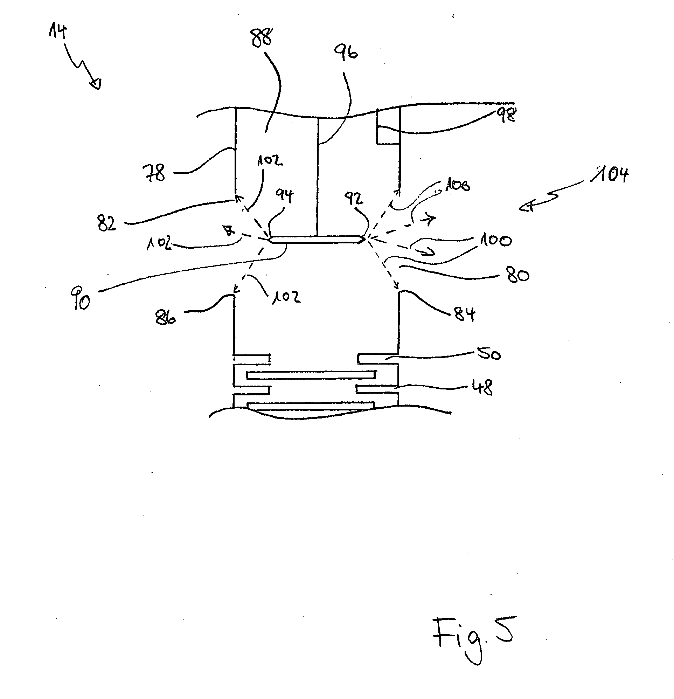

Das Ausführungsbeispiel der Vorrichtung 14, dargestellt in Fig. 5, zeigt im Wesentlichen einen vergleichbaren Aufbau zu den Vorrichtungen 10 und 12 und weist insbesondere die in der Vorrichtung 12 gezeigten Öffnungen 48 in Form der Längsschlitze 50 im Bereich des ersten Endes 42 auf. Diese Öffnungen 48 sind in einem Gehäuse 78 der Vorrichtung 14 angeordnet. Die übrigen, hier nicht näher gezeigten Bereiche des zweiten Endes 44 mit dem Griff 34 und dem Dichtungselement 26 sind im Wesentlichen identisch zu den Vorrichtungen 10 und 12 ausgestaltet.

Das Gehäuse 78 weist mit Bezug auf die Darstellung der Fig. 5 oberhalb der Öffnungen 48, das heißt, dem zweiten Ende 44 zugewandt, Öffnungen 80 und 82 auf. Diese Öffnungen 80 und 82 sind hier in einer Schnittansicht dargestellt und weisen im Übrigen eine kreisförmige Form auf. Dabei bildet sich an den jeweiligen Öffnungen 80 und 82 jeweils eine Kante 84 bzw. 86 aus.

Auch das Gehäuse 78 der Vorrichtung 14 weist einen inneren Hohlraum 88 auf. In diesem inneren Hohlraum 88 ist eine spitzförmige Elektrode 90 angeordnet. Diese spitzförmige Elektrode ist durch spitz zulaufende Enden gekennzeichnet, die jeweils Spitzen 92 und 94 aufweisen. Diese Spitzen 92, und 94 sind vorzugsweise so ausgerichtet, dass sie jeweils einen gleichmäßigen Abstand zur kreisförmig verlaufenden Kante 84 bzw. 86 haben und somit im Wesentlichen auf die Mitte der jeweiligen Öffnung 80 bzw. 82 zeigen. Die spitzförmige Elektrode 90 ist über eine Zuleitung 96 mit einer Stromquelle verbunden. Ferner verbindet eine weitere Zuleitung 98 das Gehäuse 78 ebenfalls mit der Stromquelle. Das Gehäuse 78 bildet somit ebenfalls eine Elektrode 99. Die beiden Zuleitungen 96 und 98 sind ebenfalls vorzugsweise durch den inneren Hohlraum 88 der Vorrichtung 14, bzw. des Gehäuses 78 zum zweiten Ende 44 geführt, wo sie vorzugsweise am Stromanschluss 36 im Griff 34 enden und so mit einer Stromquelle verbunden werden können. Vorliegend sind somit zwei Elektroden vorhanden, die eine wird durch die spitzförmige Elektrode 90 gebildet, während die andere Elektrode 99 durch das Gehäuse 78 der Vorrichtung 14 gebildet wird.

Wird nun eine Spannung zwischen der spitzförmigen Elektrode 90 und dem Gehäuse 78 angelegt, sprühen Elektronen von den Spitzen 92 und 94 der spitzförmigen Elektrode 90 in Richtung der Kanten 84 und 86 der Öffnungen 80 und 82. Es bildet sich somit ein Elektronenspray aus, der durch gestrichelte Pfeile 100 und 102 verdeutlicht ist. Aufgrund dieses Elektronensprays wird ebenfalls der molekulare Sauerstoff in der Luft aufgebrochen und unter anderem Ozon gebildet. Somit bilden die spitzförmige Elektrode 90 und das Gehäuse 78 als Elektrode 99 zusammen eine Ozonerzeugungseinheit 104 aus. Diese Ozonerzeugungseinheit 104 ist im inneren Hohlraum 88 des Gehäuses 78 angeordnet.

In dem Elektronenspray werden die Elektronen, und ggf. bereits gebildete Ionen und Radikale, in Richtung auf die Öffnungen 80 und 82, bzw. in Richtung auf deren Kanten 84 und 86 hin beschleunigt, woraufhin diese zum Teil auch außerhalb des inneren Hohlraumes 88 der Vorrichtung 14 gelangen. Dadurch wird eine bereits zuvor erwähnte Konvektion noch zusätzlich verstärkt. Hier nicht näher gezeigter sauerstoffhaltige Luft 68 kann durch die Längsschlitze 50, also die Öffnungen 48, in den inneren Hohlraum 88 eindringen, wird zur spitzförmigen Elektrode 90 befördert und dort aufgrund der Elektronensprays 100, 102 durch die Öffnungen 80 und 82 wieder aus dem inneren Hohlraum 88 und somit aus dem Gehäuse 78 nach draußen befördert. Dabei wird aufgrund der jeweiligen Entladungen Ozon und damit ebenfalls wieder ozonhaltige Luft 72 gebildet und in den Innenraum eines jeweiligen Behälters zur Desinfektion transportiert. Aufgrund der Bildung des Ozons, und sonstiger reaktiver Spezien, wie O- oder OH-Radikale, durch den jeweiligen Elektronenspray 100 bzw. 102 anstatt direkt an einer Elektrodenoberfläche findet keine Bildung von Belägen durch ggf. vorhandene organische Moleküle in der sauerstoffhaltigen Luft 68 an der spitzförmigen Elektrode 90 statt. Diese Anordnung ist somit sehr wartungsarm.

Sowohl die Ozonerzeugungseinheit 104 als auch die Ozonerzeugungseinheit 56 können Ozon in trockener Luft und insbesondere auch in feuchter Luft erzeugen. Dies ist zum einen gerade bei der Desinfektion von Restfeuchtigkeit enthaltenen Behältern, wie z.B. von Holzfässern, von Vorteil und eröffnet zum anderen auch die Möglichkeit zur gleichzeitigen Bereitstellung von Wasserstoffperoxid. Diese letztere Möglichkeit wird im Folgenden noch näher erläutert.

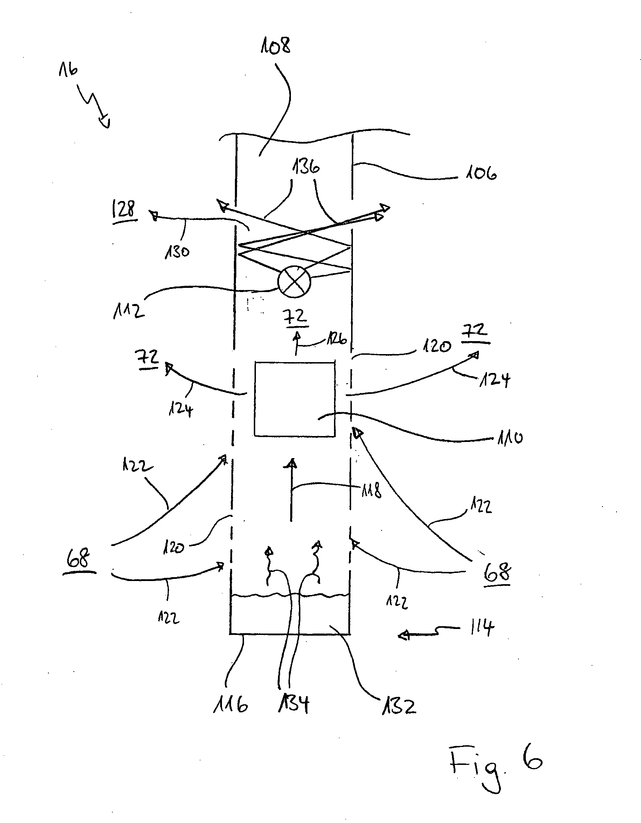

Das Ausführungsbeispiel der Vorrichtung 16, gezeigt in Fig. 6, weist ebenfalls einen zu den Vorrichtungen 10 und 12 vergleichbaren Gesamtaufbau auf. Hierbei kann wieder der nicht gezeigte Teil des zweiten Endes als im Wesentlichen identisch zu den Vorrichtungen 10 und 12 angesehen werden. Im Übrigen weist die Vorrichtung 16 ein Gehäuse 106 auf, das einen inneren Hohlraum 108 hat. In diesem inneren Hohlraum ist neben einer hier nur schematisch dargestellten Ozonerzeugungseinheit 110 eine UV-Lichtquelle 112 vorgesehen. Die Ausführungsform der Ozonerzeugungseinheit 110 kann je nach Bedarf gewählt werden und ist vorzugsweise eine der zuvor genannten Ozonerzeugungseinheiten 56 oder 104. Ferner weist das Gehäuse 106 ein erstes Ende 114 auf, das durch einen Deckel 116 verschlossen ist. Sowohl die Ozonerzeugungseinheit 110 als auch die UV-Lichtquelle 112 erzeugen Wärme, wodurch ebenfalls wieder eine Konvektion von Luft entsteht. Diese Luftkonvektion innerhalb des Gehäuses 106 ist hier in Fig. 6 durch einen Pfeil 118 angedeutet. Das Gehäuse 106 der Vorrichtung 16 weist im Bereich des ersten Endes 114 ebenfalls eine Mehrzahl von Öffnungen 120 auf. Diese Öffnungen sind hier in der Darstellung der Fig. 6 lediglich schematisch angedeutet. Dabei kann sowohl eine zur Vorrichtung 10 vergleichbare Anordnung von Rundlöchern 30, als auch Längsschlitze 50 entsprechend der Vorrichtung 12 in der Vorrichtung 16 vorgesehen werden.

Durch diese Öffnungen 120 strömt sauerstoffhaltige Luft 68 aufgrund der Konvektion 118 in den inneren Hohlraum 108 des Gehäuses 106. Dies ist durch Pfeile 122 schematisch angedeutet. Die sauerstoffhaltige Luft 68 wird dann an der Ozonerzeugungseinheit 110 zu ozonhaltiger Luft 72 umgesetzt. Diese strömt dann, wie durch Pfeile 124 angedeutet, durch weitere Öffnungen 120 wieder aus dem inneren Hohlraum 108 des Gehäuses 106 nach außen. Daneben strömt auch weitere ozonhaltige Luft 72 innerhalb des inneren Hohlraums 108 entsprechend der Konvektion 118 in Richtung der UV-Lichtquelle 112. Dies ist durch einen weiteren Pfeil 126 angedeutet. Diese ozonhaltige Luft 72 im inneren Hohlraum 108 kann nun in Anwesenheit von Luftfeuchtigkeit, das heißt in Anwesenheit von Wasser bzw. Wasserdampf, und unter Einwirkung der UV-Strahlung der UV-Lichtquelle 112 zu einer Bildung von Wasserstoffperoxid führen. Diese dann entstandene wasserstoffperoxidhaltige Luft 128 kann dann ebenfalls durch Öffnungen 120 auch nach außen treten. Dies ist durch einen weiteren Pfeil 130 angedeutet.

Da Wasserstoffperoxid sich ebenfalls sehr gut als Desinfektionsmittel eignet, ist es in einer bevorzugten Ausführungsform vorgesehen, dass die Bildung von Wasserstoffperoxid begünstigt wird. Dazu wird zusätzlich Wasser, vorzugsweise destilliertes Wasser 132, im Bereich des ersten Endes 114 im inneren Hohlraum 108 des Gehäuses 106 vorgesehen. Durch die zuvor beschriebenen Konvektion 118 und das Einströmen der sauerstoffhaltigen Luft 68 entsprechend der Pfeile 122 bewegt sich über diesem destillierten Wasser 132 gebildeter Wasserdampf in Richtung der Ozonerzeugungseinheit 110 und der UV-Lichtquelle 112. Dies ist durch Pfeile 134 zusätzlich angedeutet. Eine Begünstigung der Bildung des Wasserdampfes aus dem destillierten Wasser 132 kann zusätzlich durch die Wärmeerzeugung der Ozonerzeugungseinheit 110 und der UV-Lichtquelle 112 erfolgen. Ferner kann auch eine separate Wärmequelle oder ein sonstiges Dampf erzeugendes Element vorgesehen sein, um das destillierte Wasser 132 gezielt zu verdampfen.

Neben der Bildung von Wasserstoffperoxid innerhalb des inneren Hohlraums 108 des Gehäuses 106 erfolgt diese auch außerhalb des Gehäuses 106. Hierzu treten UV-Lichtstrahlen 136, wie durch die entsprechenden Pfeile angedeutet, durch die Öffnungen 120 nach außen. Hierzu sind in dem gezeigten Ausführungsbeispiel der Vorrichtung 16 keine Öffnungen 120 auf Höhe der UV-Lichtquelle 112 vorgesehen, so dass zunächst eine Reflektion der UV-Lichtstrahlen 136 an der Innenwand des Gehäuses 106 stattfindet und somit eine Verstärkung der UV-Lichtstahlen 136 eintritt. Aus dem Gehäuse 106 ausgetreten, können diese UV-Lichtstrahlen 136 dann ebenfalls dafür sorgen, dass außerhalb des Gehäuses 106 vorhandenes Ozon mit Wasser zu Wasserstoffperoxid reagiert. Ferner begünstigt die UV-Lichtstrahlung in Form der Lichtstrahlen 136 die Bildung von Kettenreaktionen, die durch UV-Licht induziert und aufrechterhalten werden können, und somit auch den Abbau von innerhalb eines zu desinfizierenden Behälters vorhandenen organischen Verbindungen. Dies liefert somit einen weiteren Beitrag zur Desinfektion und zur Beseitigung von unerwünschten und schädlichen Gerüchen und Geschmacksstoffen.

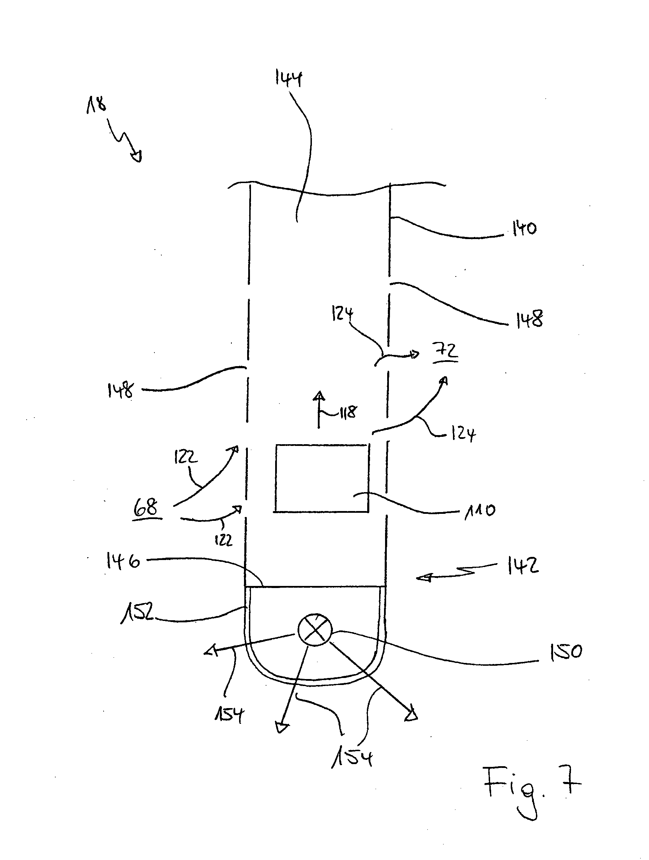

Das Ausführungsbeispiel der Vorrichtung 18 in Fig. 7 weist abermals einen vergleichbaren Aufbau zu den Vorrichtungen 10 und 12 der vorliegenden Erfindung auf. Dies bedeutet, dass insbesondere der hier nicht gezeigte Bereich des zweiten Endes mit dem Griff und dem Dichtungselement identisch zu diesen Vorrichtungen ausgestaltet ist.

Die Vorrichtung 18 weist ferner ein Gehäuse 140 auf, welches ein erstes Ende 142 und einen inneren Hohlraum 144 aufweist. Der innere Hohlraum 144 ist am ersten Ende 142 mittels eines Deckels 146 verschlossen. Das Gehäuse 140 weist ferner Öffnungen 148 auf, die ebenso wie bei der Vorrichtung 16 als Rundlöcher 30 oder Langschlitze 52 vergleichbar zu den Ausführungsformen der Vorrichtungen 10 und 12 ausgestaltet sein können. Ferner ist im Bereich des ersten Endes 142 eine Ozonerzeugungseinheit 110 vorgesehen, die wie auch bei der Vorrichtung 16 entsprechend einer der zuvor erläuterten Ausführungsformen für Ozonerzeugungseinheiten 56 oder 104 aus den Vorrichtungen 12 und 14 ausgestaltet sein kann. Aufgrund der Wärmeentwicklung bei der Ozonerzeugung in der Ozonerzeugungseinheit 110 entsteht ebenfalls wieder eine Konvektion 118, wie dies zuvor bereits im Zusammenhang mit der Vorrichtung 16 erläutert wurde. Dadurch strömt sauerstoffhaltige Luft 68 entsprechend der Pfeile 122 in die Öffnungen 148 des Gehäuses 140 ein. In dem inneren Hohlraum 144 wird dann an der Ozonerzeugungseinheit 110 ozonhaltige Luft 72 erzeugt und aufgrund der Konvektion entsprechend der Pfeile 124 durch die Öffnungen 148 wieder aus dem Gehäuse 140 nach außen geführt.

Zusätzlich weist die Vorrichtung 18 am ersten Ende 142 eine UV-Lichtquelle 150 auf. Diese UV-Lichtquelle ist in dem vorliegenden Ausführungsbeispiel durch eine UV-lichtdurchlässige Kappe 152 schützend umgeben. Durch diese UV-Lichtquelle 150 wird UV-Licht in Form von UV-Lichtstrahlen 154 nach außen in den Innenraum eines entsprechenden zu desinfizierenden Behälters gestrahlt. Durch vorhandene Feuchtigkeit in der Luft und allgemein im Innenraum des zu desinfizierenden Behälters kann es nun durch die Zufuhr von ozonhaltiger Luft 72 abermals zur Bildung von Wasserstoffperoxid unter der Einwirkung der UV-Lichtstrahlen 154 kommen. Ferner besteht auch hier die Möglichkeit, dass in dem Innenraum des jeweiligen Behälters durch die UV-Lichtstrahlen 154 Kettenreaktionen gestartet und/oder aufrechterhalten werden können. Somit findet auch hier eine gründliche Desinfektion und Reinigung im Hinblick auf unerwünschte organische Verbindungen, wie z.B. Geruchs- oder Geschmacksstoffe, statt. Im Unterschied zu dem Ausführungsbeispiel der Vorrichtung 16 findet hier die Erzeugung von Wasserstoffperoxid und ähnlichen Verbindungen ausschließlich im Innenraum des Behälters statt. Demgegenüber wird in der Vorrichtung 16 auch im inneren Hohlraum 108 Wasserstoffperoxid erzeugt und so der Umgebungsluft im Innenraum des entsprechenden Behälters als wasserstoffperoxidhaltige Luft 128 zugeführt.

Neben der Erzeugung von Wasserstoffperoxid können die UV-Lichtquellen 112 und 150 der Vorrichtungen 16 und 18 außerdem dazu eingesetzt werden, nach Abschalten der jeweiligen Ozonerzeugungseinheit 110 für einen beschleunigten Abbau, das heißt für eine schnellere Zersetzung des restlichen Ozons im Innenraum des Behälters, zu sorgen. Dies ist auch entsprechend übertragbar auf die Vorrichtungen 10, 12 und 14, wenn diese mit UV-Lichtquellen modifiziert werden.

Im Zusammenhang mit den Figuren 8 und 9 und den zuvor gemachten Erläuterungen soll nun die Funktionsweise und entsprechend auch das erfindungsgemäße Verfahren näher erläutert werden.

In Fig. 8 ist dazu ein Holzbehälter in Form eines Holzfasses 160 gezeigt. Dieses Holzfass 160 weist an seinem oberen Ende, also am Deckel eine Öffnung 162 auf. Diese Öffnung 162 ist üblicherweise als eine runde Öffnung ausgestaltet. In dieses Holzfass 160 ist die erfindungsgemäße Vorrichtung 12 durch die Öffnung 162 eingeführt.

Neben der hier gezeigten exemplarischen Ausgestaltung des Holzfasses 160, bei dem die Öffnung 162 mittig im Deckel angeordnet ist, kommen auch andere Ausgestaltungen von Holzfässern in Frage, bei denen die jeweilige Öffnung z.B. in einer Fassdaube vorliegt. Dabei können auch zwei oder mehr Öffnungen vorliegen, wobei die Öffnungen, die keine erfindungsgemäße Vorrichtung aufnehmen, dann ggf. verschlossen werden, wie dies im Folgenden noch erläutert wird. Ferner kann auch, sowohl im Deckel als auch in der Fassdaube die Position der jeweiligen Öffnung variieren, so dass diese nicht zwangsläufig, wie hier zum Zwecke der Erläuterung gezeigt, mittig angeordnet sein muss. Die nachfolgenden Erläuterungen im Zusammenhang mit Holzfass 160 sind entsprechend auf die zuvor genannten und andere Fässer mit alternativen Bauweisen übertragbar. Die Funktionsweise der erfindungsgemäßen Vorrichtung und das erfindungsgemäße Verfahren sind somit nicht an die gezeigte Form und Ausgestaltung des Holzfasses 160 gebunden.

Wenngleich auch hier und im Folgenden die Erläuterungen der Funktionsweise der erfindungsgemäß Vorrichtungen und des erfindungsgemäßen Verfahrens anhand der Vorrichtung 12 vorgenommen werden, so ist klar, dass die entsprechend gemachten Aussagen ebenfalls auf die Vorrichtungen 10, 14, 16 und 18 in analoger Weise übertragbar sind.

Anhand der Darstellung der Figuren 8 und 9 ist ersichtlich, dass die Öffnung 162 die Vorrichtung 12 an dem Dichtungselement 26 aufnimmt. Dadurch kommt das zweite Ende 44 mit dem Griff 34 und dem Anschluss 36 außerhalb des Behälters, also des Holzfasses 160, zu liegen. Demgegenüber befindet sich der übrige Teil mit dem ersten Ende 42 und den Öffnungen 48 sowie der Ozonerzeugungseinheit 56, die im inneren Hohlraum 54 des Gehäuses 40 angeordnet ist, im Innenraum 164 des Holzfasses 160. Da das Holzfass 160 keine übrigen Öffnungen aufweist oder diese vorzugsweise verschlossen sind, ist der Innenraum 164 und damit auch das Holzfass 160 verschlossen.

Sofern kein oder kein nennenswerter gezielter Gasaustausch vorgenommen wurde, findet sich im Innenraum 164 des Fasses 160 sauerstoffhaltige Luft 68.

Am Stromanschluss 36 ist ein Kabel 166 angeordnet, das wiederum mit einer Stromquelle 167 verbunden ist und über das Strom entlang der Kabel 76 zu der Ozonerzeugungseinheit 56 geführt wird. Diese Zuführung des Stroms ist ferner durch einen Pfeil 168 in Fig. 9 angedeutet.

Wird diese Stromzuführung 168 eingeleitet, findet an der Ozonerzeugungseinheit 56 eine entsprechende Entladung und ein entsprechender Stromfluss statt, so dass sich die Keramikhälfte 60, also das Dielektrikum, erwärmt. Dadurch kommt es, genau wie in allen anderen gezeigten Ausführungsbeispielen von Ozonerzeugungseinheiten 104 und 110, zu der genannten und bereits erläuterten Konvektion. Durch die Entladung an der Ozonerzeugungseinheit 56 wird Ozon innerhalb des Holzfasses 160, das heißt im Innenraum 164, bereitgestellt. Diese Bereitstellung geschieht erfindungsgemäß dadurch, dass dieses Ozon in dem Innenraum 164 erzeugt wird.

Durch die genannte Konvektion strömt nun, angedeutet durch Pfeile 70, sauerstoffhaltige Luft 68 in den inneren Hohlraum 54 des Gehäuses 40. Diese sauerstoffhaltige Luft 68 tritt dabei durch die Öffnungen 48 in den inneren Hohlraum 54 ein und wird dabei in die Entladungszone 66 geleitet, wie dies bereits z.B. im Zusammenhang mit der Fig. 4 erläutert wurde. Dort kommt es dann durch die Ozonerzeugungseinheit 56 zu der gewünschten Ozonerzeugung. Die dadurch gebildete ozonhaltige Luft 72 wird dann, wie dies durch Pfeile 74 angedeutet ist, wieder durch die Öffnungen 48 aus dem inneren Hohlraum 54 in den Innenraum 164 des Fasses 160 geleitet. In dem Innenraum 164 wird dann aufgrund der Konvektion und/oder vermittelt durch Diffusion eine Verteilung dieser ozonhaltigen Luft 72 erreicht. Dies ist durch Pfeile 170 angedeutet. Dadurch kann sich die ozonhaltige Luft 72 und damit das Ozon im gesamten Innenraum 164 verteilen und erreicht somit die gesamte innere Oberfläche des Holzfasses 160. Hierbei kann das Ozon sogar in das Holz des Holzfasses 160 vom Innenraum 164 her eindringen. Diese Wirkung des Ozons auf die Innenwände des Holzfasses 160 ist ebenfalls durch Pfeile 172 angedeutet. Das Ergebnis ist eine Desinfektion des gesamten Innenraums 164 des Holzfasses 160, sowohl hinsichtlich aerober als auch anaerober Mikroorganismen. Ferner werden unerwünschte organische Substanzen, wie Geruchs- oder Geschmacksstoffe beseitigt.

Die Anwendung dieser erfindungsgemäßen Vorrichtungen 10, 12, 14, 16 und 18 erfolgt dadurch, dass die Vorrichtung jeweils bspw. am Griff 34 gefasst und in eine Öffnung 162 des Holzbehälters, hier des Holzfasses 160, eingeführt wird. Dadurch wird ein Verschließen des Holzbehälters aufgrund des Dichtungselements 26 erreicht. Durch Stromzufuhr, angedeutet durch den Pfeil 168, wird dann durch die Ozonerzeugungseinheit, z.B. der Ozonerzeugungseinheit 56 oder 104, Ozon erzeugt. Durch die kontinuierliche Zufuhr von Strom erfolgt somit auch die Ozonerzeugung kontinuierlich. Entsprechend ist der durch die Pfeile 70, 74, 170 und 172 dargestellte Luftverlauf und damit auch die Desinfektionswirkung ebenfalls als kontinuierlich anzusehen. Ist die Desinfektion oder Reaktion des Ozons abgeschlossen und ist dieses wieder zu Sauerstoff abreagiert, liegt damit wieder sauerstoffhaltige Luft 68 vor, die in einem nächsten Zyklus, der mit dem Pfeil 70 beginnt, eintreten kann. Soll dieses Abreagieren zu Sauerstoff nach beendeter Desinfektion beschleunigt werden, kann eine Bestrahlung des Innenraums des Holzbehälters mit UV-Licht nach Abschalten der Ozonerzeugungseinheit vorgesehen werden.

Wie insbesondere im Zusammenhang mit den Figuren 6 und 7, das heißt mit den Vorrichtungen 16 und 18, erläutert wurde, kann zusätzlich eine UV-Lichtquelle 112 bzw. 150 vorgesehen werden. Neben der zuvor erwähnten Ozonbeseitigung kann dadurch eine Erzeugung von Wasserstoffperoxid begünstigt werden, welches ebenfalls an der Oberfläche und allgemein im Innenraum 164 eines Holzfasses 160 zu einer Desinfektionswirkung führen kann. Um dies weiter zu begünstigen, kann entsprechend der Darstellung in Fig. 6 und der Erläuterungen im Zusammenhang mit der Vorrichtung 16 zusätzlich (destilliertes) Wasser 132 vorgesehen werden, so dass der entsprechende Wasserdampf 134 bereits bei der Ozonerzeugung vorliegen kann. Die Wirkungen der dadurch erhaltenen wasserstoffperoxidhaltigen Luft 128 sind dann vergleichbar mit und analog zu den Erläuterungen im Zusammenhang mit der Fig. 9 und der ozonhaltigen Luft 72.

Sowohl wasserstoffperoxidhaltige Luft 128 als auch ozonhaltige Luft 72 reagieren mit der Zeit rückstandsfrei ab und stellen somit, nach Abschalten der Ozonerzeugung, keine gesundheitliche Gefahr mehr dar.

## Patentansprüche

1. Vorrichtung zur Desinfektion eines Innenraums (164) eines Behälters (160), mit
- einem stabförmigen Gehäuse (20, 40, 78, 106, 140), mit einem ersten Ende (22, 42, 114, 142) zur Anordnung innerhalb des Behälters (160), einem gegenüberliegenden zweiten Ende (24, 44) und einem inneren Hohlraum (54, 108, 144). **gekennzeichnet durch**
- eine, Ozonerzeugungseinheit (56, 104, 110) im inneren Hohlraum (54, 108, 144), und
- zumindest einer Mehrzahl von Öffnungen (28, 48, 120, 148) im Gehäuse (20, 40, 78, 106, 140) im Bereich des ersten Endes (22, 42, 114, 142),
wobei das Gehäuse (20, 40, 78, 106, 140) an seinem zweiten Ende (24, 44) außen ein Dichtungselement (26) aufweist, **durch** das die Vorrichtung dichtend in einer Öffnung (162) des Behälters (160) aufgenommen werden kann.

2. Vorrichtung nach Anspruch 1, **dadurch gekennzeichnet, dass** das Gehäuse (20, 40, 78, 106, 140) einen runden Querschnitt aufweist.

3. Vorrichtung nach Anspruch 1 oder 2, **dadurch gekennzeichnet, dass** das Gehäuse (20, 40, 78, 106, 140) aus Metall ist.

4. Vorrichtung nach einem der Ansprüche 1 bis 3, **dadurch gekennzeichnet, dass** die Mehrzahl von Öffnungen im Gehäuse (20, 40, 78, 106, 140) als Rundlöcher (30) ausgestaltet sind.

5. Vorrichtung nach einem der Ansprüche 1 bis 3, **dadurch gekennzeichnet, dass** die Mehrzahl von Öffnungen (28, 48, 120, 148) im Gehäuse (20, 40, 78, 106, 140) als Längsschlitze (50) ausgestaltet sind.

6. Vorrichtung nach einem der Ansprüche 1 bis 5, **dadurch gekennzeichnet, dass** die Ozonerzeugungseinheit (56, 110) eine Induktionselektrode (62) und eine Entladungselektrode (64) aufweist, die durch ein festes Dielektrikum (60) voneinander getrennt sind.

7. Vorrichtung nach einem der Ansprüche 1 bis 5, **dadurch gekennzeichnet, dass** die Ozonerzeugungseinheit (104, 110) eine erste spitzförmige Elektrode (90) und eine zweite Elektrode (99) aufweist, wobei die zweite Elektrode (99) durch das Gehäuse (20, 40, 78, 106, 140) gebildet wird und das Gehäuse (20, 40, 78, 106, 140) in Längsrichtung gesehen auf Höhe der spitzförmigen Elektrode zumindest eine Öffnung (80, 82) aufweist.

8. Vorrichtung nach einem der Ansprüche 1 bis 7, **dadurch gekennzeichnet, dass** die Vorrichtung ferner eine UV-Lichtquelle (112; 150) aufweist, die im Gehäuse (20, 40, 78, 106, 140) und/oder außen am Gehäuse (20, 40, 78, 106, 140) angeordnet ist.

9. Vorrichtung nach einem der Ansprüche 1 bis 8, **dadurch gekennzeichnet, dass** das Gehäuse (20, 40, 78, 106, 140) insbesondere im Bereich des ersten Endes (22, 42, 114, 142) zumindest teilweise aus einem inerten Material, insbesondere aus Edelstahl und/oder Kunststoff und/oder Aluminium und/oder Silber und/oder Palladium, besteht.

10. Vorrichtung nach einem der Ansprüche 1 bis 9, **dadurch gekennzeichnet, dass** das Gehäuse (20, 40, 78, 106, 140) im Bereich des ersten Endes (22, 42, 114, 142) zumindest teilweise ein Metalloxidmaterial aufweist, wobei vorzugsweise das Gehäuse im Bereich des ersten Endes eine metalloxidische Beschichtung oder einen metalloxidischen Überzug aufweist, insbesondere eine oxidische Beschichtung mit Aluminiumoxid und/oder Kupferoxid und/oder Manganoxid.

11. Verfahren zur Desinfektion des Innenraums (164) von Holzbehältern (160) durch Bereitstellen von Ozon im Holzbehälter (160), wobei das Ozon im Holzbehälter (160) erzeugt wird und wobei das Verfahren die folgenden Schritte aufweist:
- Anordnen einer Ozonerzeugungseinheit nach Anspruch 1 (56, 104, 110) im Holzbehälter (160),
- Verschließen des Holzbehälters (160),
- Erzeugen von Ozon durch die Ozonerzeugungseinheit (56, 104, 110).

12. Verfahren nach Anspruch 11, **dadurch gekennzeichnet, dass** das Erzeugen von Ozon kontinuierlich erfolgt.

13. Verfahren nach Anspruch 11 oder Anspruch 12, **dadurch gekennzeichnet, dass** der Innenraum (164) des Holzbehälters (160) ferner UV-Licht ausgesetzt wird.

14. Verfahren nach Anspruch 13, **dadurch gekennzeichnet, dass** im Innenraum (164) ferner Wasserdampf (134) bereitgestellt wird.

## Claims

1. A device for disinfecting an interior (164) of a container (160), comprising
- a rod-shaped housing (20, 40, 78, 106, 140) having a first end (22, 42, 114, 142) for placement within the container (160), an opposite second end (24, 44), and an inner hollow space (54, 108, 144),
**characterized by**
- an ozone-generating unit (56, 104, 110) in the inner hollow space (54, 108, 144), and
- at least a plurality of openings (28, 48, 120, 148) in the housing (20, 40, 78, 106, 140) in the region of the first end (22, 42, 114,142),
wherein the housing (20, 40, 78, 106, 140) at its second end (24, 44) has a sealing element (26) on the outside, by means of which the device can be accommodated, in a sealing manner, in an opening (162) of the container (160).

2. The device according to claim 1, **characterized in that** the housing (20, 40, 78, 106, 140) has a round cross-section.

3. The device according to claim 1 or 2, **characterized in that** the housing (20, 40, 78, 106, 140) is made of metal.

4. The device according to any one of claims 1 to 3, **characterized in that** the plurality of openings in the housing (20, 40, 78, 106, 140) are designed as round holes (30).

5. The device according to any one of claims 1 to 3, **characterized in that** the plurality of openings (28, 48, 120, 148) in the housing (20, 40, 78, 106, 140) are designed as elongated slots (50).

6. The device according to any one of claims 1 to 5, **characterized in that** the ozone-generating unit (56, 110) comprises an induction electrode (62) and a discharge electrode (64), which are separated from one another by a solid dielectric (60).

7. The device according to any one of claims 1 to 5, **characterized in that** the ozone-generating unit (104, 110) has a first pointed electrode (90) and a second electrode (99), wherein the second electrode (99) is formed by the housing (20, 40, 78, 106, 140), and the housing (20, 40, 78, 106, 140), as viewed in the longitudinal direction, has at least one opening (80, 82) at the level of the pointed electrode.

8. The device according to any one of claims 1 to 7, **characterized in that** the device further comprises a UV light source (112; 150), which is disposed in the housing (20, 40, 78, 106, 140) and/or on the outside of the housing (20, 40, 78, 106, 140).

9. The device according to any one of claims 1 to 8, **characterized in that** the housing (20, 40, 78, 106, 140) is formed, in particular in the region of the first end (22, 42, 114, 142), at least partially from an inert material, in particular from stainless steel and/or plastic and/or aluminium and/or silver and/or palladium.

10. The device according to any one of claims 1 to 9, **characterized in that** the housing (20, 40, 78, 106, 140) has a metal oxide material, at least partially, in the region of the first end (22, 42, 114, 142), wherein the housing preferably has, in the region of the first end, a metal-oxidic coating or a metal-oxidic covering, in particular an oxidic coating having aluminum oxide and/or copper oxide and/or manganese oxide.

11. A method for disinfecting the interior (164) of wooden containers (160) by providing ozone in the wooden container (160), wherein the ozone is generated in the wooden container (160), and wherein the method has the following steps of:
- arranging an ozone-generating unit according to claim 1 (56, 104, 110) in the wooden container (160),
- closing the wooden container (160),
- generating ozone by means of the ozone-generating unit (56, 104, 110).

12. The method according to claim 11, **characterized in that** ozone is continuously generated.

13. The method according to claim 11 or claim 12, **characterized in that** the interior (164) of the wooden container (160) is furthermore exposed to UV light.

14. The method according to claim 13, **characterized in that** furthermore water vapor (134) is provided in the interior (164).

## Revendications

1. Dispositif destiné à la désinfection de la partie intérieure (164) d'un récipient (160) avec
- un boîtier en forme de tige (20, 40, 78, 106, 140), avec une première extrémité (22, 42, 114, 142) pour la mise en place à l'intérieure du récipient (160), une deuxième extrémité (24, 44) située en face et un espace creux interne (54, 108, 144),
**caractérisé par**
- une unité de génération d'ozone (56, 104, 110) dans l'espace creux interne (54, 108, 144), et
- au moins une multiplicité d'orifices (28, 48, 120, 148) dans le boîtier (20, 40, 78, 106, 140) dans la partie de sa première extrémité (22, 42, 114, 142),
dans lequel le boîtier (20, 40, 78, 106, 140) présente un élément d'étanchéité (26) à l'extérieur de la deuxième extrémité (24, 44), par lequel le dispositif peut être admis de manière étanche dans un orifice (162) du récipient (160).

2. Dispositif selon la revendication 1, **caractérisé en ce que** le boîtier (20, 40, 78, 106, 140) présente une section transversale circulaire.

3. Dispositif selon les revendications 1 ou 2, **caractérisé en ce que** le boîtier (20, 40, 78, 106, 140) est en métal.

4. Dispositif selon l'une des revendications 1 à 3, **caractérisé en ce que** la multiplicité des orifices dans le boîtier (20, 40, 78, 106, 140) est conçue sous la forme de trous circulaires (30).

5. Dispositif selon l'une des revendications 1 à 3, **caractérisé en ce que** la multiplicité des orifices (28, 48, 120, 148) dans le boîtier (20, 40, 78, 106, 140) est conçue sous la forme de fentes allongées (50).

6. Dispositif selon l'une des revendications 1 à 5, **caractérisé en ce que** l'unité génératrice d'ozone (56, 110) présente une électrode d'induction (62) et une électrode de décharge (64), qui sont séparées l'une de l'autre par un diélectrique solide (60).

7. Dispositif selon l'une des revendications 1 à 5, **caractérisé en ce que** l'unité génératrice d'ozone (104, 110) présente une première électrode en forme de pointe (90) et une deuxième électrode (99), où la deuxième électrode (99) est formée par le boîtier (20, 40, 78, 106, 140) et le boîtier (20, 40, 78, 106, 140), vu dans le sens longitudinal, présente au moins un orifice (80, 82) de la hauteur de l'électrode en forme de pointe.

8. Dispositif selon l'une des revendications 1 à 7, **caractérisé en ce que** le dispositif présente en outre une source de lumière UV (112 ; 150) qui est disposée dans le boîtier (20, 40, 78, 106, 140) et/ou sur le boîtier (20, 40, 78, 106, 140) à l'extérieur.

9. Dispositif selon l'une des revendications 1 à 8, **caractérisé en ce que** le boîtier (20, 40, 78, 106, 140) est constitué d'un matériau inerte, en particulier en acier inoxydable, et/ou en matière synthétique, et/ou en aluminium, et/ou en argent, et/ou en palladium, notamment au moins partiellement dans la partie de sa première extrémité (22, 42, 114, 142).

10. Dispositif selon l'une des revendications 1 à 9, **caractérisé en ce que** le boîtier (20, 40, 78, 106, 140) présente au moins partiellement un matériau à base d'oxyde métallique, dans la partie de sa première extrémité (22,42, 114, 142), où, de préférence, le boîtier présente un revêtement à base d'oxyde métallique ou une enveloppe à base d'oxyde métallique dans la partie de sa première extrémité, notamment, un revêtement à base d'oxyde avec de l'oxyde d'aluminium, et/ou d'oxyde de cuivre, et/ou d'oxyde de manganèse.

11. Procédé destiné à la désinfection de la partie intérieure (164) de récipients en bois (160) par la fourniture d'ozone dans le récipient en bois (160), où l'ozone est généré dans le récipient en bois (160) et où le procédé présente les étapes suivantes :
- mise en place d'une unité de génération d'ozone (56, 104, 110) selon la revendication 1 dans le récipient en bois (160),
- fermeture du récipient en bois (160)
- génération d'ozone par l'unité de génération d'ozone (58, 104, 110).

12. Procédé selon la revendication 11, **caractérisé en ce que** la génération d'ozone est effectuée de manière continue.

13. Procédé selon la revendication 11 ou la revendication 12, **caractérisé en ce que** la partie intérieure (164) du récipient en bois (160) est en outre soumise à une lumière UV.

14. Procédé selon la revendication 13, **caractérisé en ce qu'**en outre de la vapeur d'eau (134) est préparée dans la partie intérieure (164).
